(19) Europäisches Patentamt
European Patent Office
Office européen des brevets

(11) Veröffentlichungsnummer : **0 180 839**
**B1**

(12) # EUROPÄISCHE PATENTSCHRIFT

(45) Veröffentlichungstag der Patentschrift : **02.03.88**

(21) Anmeldenummer : **85113313.2**

(22) Anmeldetag : **21.10.85**

(51) Int. Cl.⁴ : **C 07 C 51/60**, C 07 C 51/567, C 07 C 63/24, C 07 C 63/26, C 07 C 63/30, C 07 C 55/02, C 07 C 61/09, C 07 D307/16, C 07 D333/24

(54) **Verfahren zur Herstellung von Säurechloriden mehrbasischer Carbonsäuren sowie Zwischenprodukte hierfür.**

(30) Priorität : **02.11.84 DE 3439937**

(43) Veröffentlichungstag der Anmeldung : **14.05.86 Patentblatt 86/20**

(45) Bekanntmachung des Hinweises auf die Patenterteilung : **02.03.88 Patentblatt 88/09**

(84) Benannte Vertragsstaaten : **BE DE FR GB IT NL**

(56) Entgegenhaltungen :
**DE-A- 3 016 119**
**US-A- 3 318 950**
**US-A- 3 869 485**

(73) Patentinhaber : **BAYER AG**
**Konzernverwaltung RP Patentabteilung**
**D-5090 Leverkusen 1 Bayerwerk (DE)**

(72) Erfinder : **Stammann, Günter, Dr.**
**Silesiusstrasse 78**
**D-5000 Köln 80 (DE)**
Erfinder : **Freitag, Dieter, Dr.**
**Hasenheide 10**
**D-4150 Krefeld 1 (DE)**
Erfinder : **Bottenbruch, Ludwig, Dr.**
**Woehlerstrasse 5**
**D-4150 Krefeld 1 (DE)**
Erfinder : **Feltgen, Karlheinz, Dr.**
**Iltisweg 62**
**D-4047 Dormagen 11 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 180 839 B1

Jouve, 18, rue St-Denis, 75001 Paris, France

**Beschreibung**

Die vorliegende Erfindung betrifft ein Verfahren zur Herstellung von Säurechloriden mehrbasischer Carbonsäuren durch stufenweise Umsetzung von mehrbasischen Carbonsäuren, die mindestens zwei nicht zu einer inneren Anhydridbildung befähigte Carboxylgruppen enthalten, mit Säureanhydriden einbasischer Carbonsäuren und Phosgen, sowie Zwischenprodukte hierfür bestehend aus einer Suspension, die polymere Anhydride dieser Carbonsäuren enthält und Verfahren zur Herstellung solcher polymerer Anhydride.

Säurechloride mehrbasischer Carbonsäuren, insbesondere Dicarbonsäuredichloride, werden zur Herstellung von technisch wichtigen Polykondensaten benötigt, beispielsweise für die Umsetzung mit Bisphenol A zu Polyestern. Die Qualität der Säurechloride beeinflußt stark die Qualität und Eingenschaften der daraus hergestellten Polykondensate, z. B. deren Transparenz und Alterungsbeständigkeit (siehe z. B. EP-OS 0 050 783 und DE-OS 2 933 148). Es besteht deshalb ein Bedürfnis nach einem einfachen und wirtschaftlichen Verfahren zur Herstellung möglichst reiner Säurechloride mehrbasischer Carbonsäuren, die allenfalls geringe Mengen solcher Verunreinigungen enthalten, welche die Qualität und Eigenschaften der daraus hergestellten Polykondensate nicht negativ beeinflussen.

Es ist bekannt, daß man Terephthalsäureanhydrid mit einem maximalen Kondensationsgrad von $n = 3$ erhält, wenn man Terephthalsäure mit einem großen Überschuß von siedendem Essigsäureanhydrid behandelt, danach nicht umgesetztes Essigsäureanhydrid zuerst bei Normaldruck, dann im Vakuum entfernt, das zurückbleibende Produkt pulverisiert, es zur Entfernung von unumgesetzter Terephthalsäure mit Sodalösung stehen läßt, anschließend mit Wasser wäscht, mit konzentrierter Salzsäure stehen läßt, erneut mit Wasser wäscht und bei 100 °C trocknet (siehe F.A. Henglein und H.-E. Tarrach, Kunststoffe-Plastics 6, 5 bis 8 (1959)). Terephthalsäureanhydride mit höheren Kondensationsgraden (bis zu $n = 33$) erhält man nur dann, wenn man Terephthalsäure mit Terephthalsäuredichlorid, Terephthalsäure mit Phthalsäuredichlorid oder Terephthalsäuredichlorid mit Phthalsäure bei Temperaturen von 200 bis 300 °C umsetzt (siehe a.a.O.).

Solche Verfahren sind unbrauchbar, wenn die polymeren Terephthalsäureanhydride als Zwischenprodukte für die Herstellung von Terephthalsäuredichlorid dienen sollen. Terephthalsäureanhydride, die gemäß dieser Literaturstelle unter Verwendung von Essigsäureanhydrid hergestellt werden, weisen trotz des umständlichen und aufwendigen Verfahrens nur niedrige Kondensationsgrade auf. Sie sind deshalb zur Herstellung von möglichst reinem Terephthalsäuredichlorid nicht geeignet, da bezogen auf vorhandene —C—CO—Brücken relativ viele —COOH-Endgruppen vorliegen, die bei der Umwandlung der Terephthalsäureanhydride in Terephthalsäuredichlorid störende Nebenprodukte in größeren Mengen ergeben.

Aus « Spezialplaste » herausgegeben von Dr. Konrad-Ulrich Bühler, Akademie-Verlag Berlin (1978), Seite 360 bis 361 ist bekannt, daß man hochmolekulare Polyanhydride von z. B. Terephthalsäure erhalten kann, indem man zunächst eine aromatische Dicarbonsäure mit Essigsäureanhydrid bei 160 °C in ein gemischtes Anhydrid überführt, aus diesem durch Erhitzen auf 160 bis 200 °C unter Abspaltung von Essigsäureanhydrid ein niedermolekulares Vorpolymer gewinnt und dieses bei 220 bis 280 °C und einem Druck von 1 mm Hg (= 0,75 mbar) unter weiterer Abgabe von Essigsäureanhydrid kondensiert. Dieses Verfahren liefert zwar für die Herstellung möglichst reiner Säurechloride von mehrbasischen Carbonsäuren geeignete polymere Anhydride, jedoch ist es ebenfalls aufwendig und unwirtschaftlich, weil bei hohen Temperaturen und sehr niedrigen Drucken gearbeitet werden muß. Die technische Durchführbarkeit ist erschwert, da zum Anbacken neigende Feststoffkuchen getempert werden müssen.

In der US-PS 3 318 950 wird ein Verfahren zur Herstellung von Carbonsäurechloriden beschrieben, bei dem man intermolekulare Carbonsäureanhydride mit Phosgen unter bestimmten Reaktionsbedingungen umsetzt. Die wesentlichen dieser Reaktionsbedingungen sind die Gegenwart eines Carboxamides als Katalysator und das Vorliegen einer Anhydridverbindung einer Dicarbonsäure in flüssiger oder gelöster Form. Da sich solche Anhydridverbindungen in üblichen Lösungsmitteln nicht lösen, kommt für diese nur das jeweils hergestellte Säurechlorid als Lösungsmittel infrage. Hierbei ist die Rückführung großer Mengen des gebildeten Carbonsäurechlorids nachteilig. Außerdem werden Produkte mit Verunreinigungen erhalten, die sich bei der weiteren Verarbeitung zu Polykondensaten negativ auswirken.

Es wurde nun ein Verfahren zur Herstellung von Säurechloriden mehrbasischer Carbonsäuren gefunden, das dadurch gekennzeichnet ist, daß man zunächst aus mehrbasischen Carbonsäuren, die mindestens zwei nicht zu einer inneren Anhydridbildung befähigte Carboxylgruppen enthalten, durch Umsetzung mit Anhydriden einbasischer $C_2$- bis $C_4$-Carbonsäuren polymere Anhydride mehrbasischer Carbonsäuren herstellt, wobei man vor oder während dieser Reaktion ein Lösungsmittel zusetzt, in dem die Anhydride einbasischer $C_2$- bis $C_4$-Carbonsäuren löslich sind, das höher siedet als diese und in dem die polymeren Anhydride mehrbasischer Carbonsäuren unlöslich sind und anschließend in die nach Abtrennung niedriger als das Lösungsmittel siedender Bestandteile erhaltene Suspension Phosgen in Gegenwart von Katalysatoren einleitet.

Im folgenden Schema wird das erfindungsgemäße Verfahren am Beispiel der Herstellung von Terephthalsäuredichlorid aus Terephthalsäure, Essigsäureanhydrid und Phosgen erläutert.

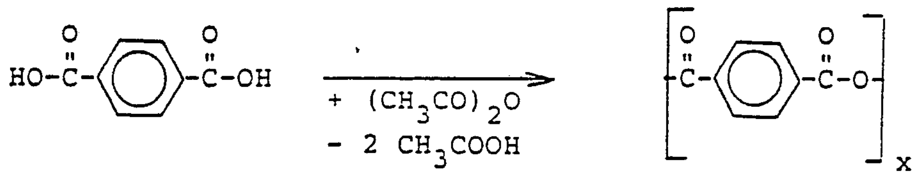


(1. Stufe)

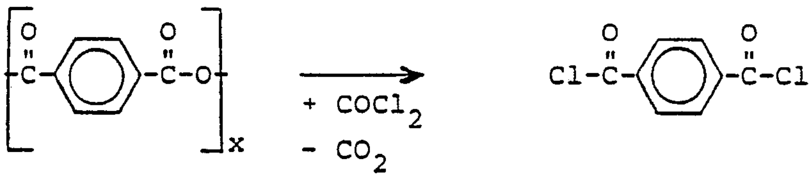


(2. Stufe)

In das erfindungsgemäße Verfahren werden mehrbasische Carbonsäuren eingesetzt, die mindestens zwei nicht zu innerer Anhydridbildung befähigte Carboxylgruppen enthalten. Diese mehrbasischen Carbonsäuren können beispielsweise ein aliphatisches, cycloaliphatisches, heterocyclisches, aromatisches oder araliphatisches Gerüst enthalten, das beispielsweise, ohne Berücksichtigung der Carboxylgruppen, 4 bis 50 C-Atome, vorzugsweise 4 bis 15 C-Atome enthalten kann. Die Carboxylgruppen tragenden Molekülgerüste können anellierte und/oder kondensierte Ringe oder Ringsysteme enthalten, welche auch über, gegebenenfalls Heteroatome, wie O, S, N oder P, enthaltende Brückenglieder verbunden sein können, und sie können weiter substituiert sein, beispielsweise mit $C_1$- bis $C_{10}$-Alkyl-, Trifluormethyl-, Cyclohexyl-, Phenyl-, 4-Chlorphenyl-, 3-Trifluormethylphenyl-, Methoxy- und/oder Phenoxygruppen.

Die mehrbasischen Carbonsäuren können neben den zwei nicht zur inneren Anhydridbildung befähigten Carboxylgruppen weitere Carboxylgruppen beliebiger Anordnung enthalten. Sie können pro Molekül insgesamt beispielsweise 2 bis 5 Carboxylgruppen enthalten. Vorzugsweise werden solche mehrbasischen Carbonsäuren eingesetzt, die außer zwei nicht zu innerer Anhydridbildung befähigten Carboxylgruppen keine weiteren Carboxylgruppen enthalten.

Beispiele für das erfindungsgemäße Verfahren geeigneter mehrbasischer Carbonsäuren sind Adipinsäure, Sebacinsäure, cis-1,4-Cyclohexandicarbonsäure, trans-1,4-Cyclohexandicarbonsäure, Trimesinsäure, Isophthalsäure, Terephthalsäure, 2,5-Furandicarbonsäure, sowie Dicarbonsäuren, die den Formeln (I) bis (VI) entsprechen.

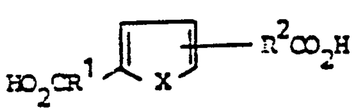

(I)

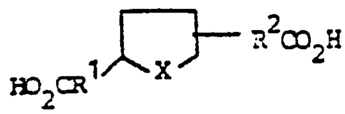

(II)

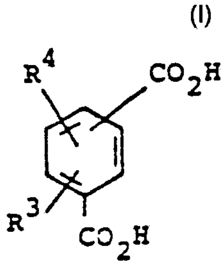

(III)

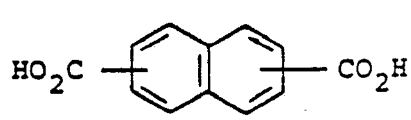

(IV)

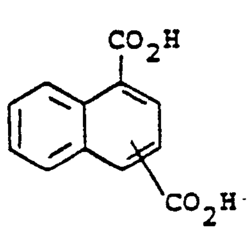

(V)

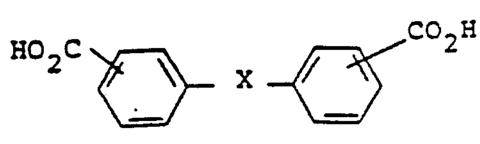

(VI)

In den Formeln (I) bis (VI) bedeutet

X Sauerstoff, Schwefel oder die Gruppe $-N(CH_3)-$,

$R^1$ und $R^2$ unabhängig voneinander eine Einfachbindung, eine $C_1$- bis $C_6$-Alkylengruppe, eine $-CO-$ oder $-SO_2$-Gruppe oder die Gruppe $-X_1-(CH_2)_m-X_2-(CH_2)_n-X_3-$, in der $X_1$, $X_2$ und $X_3$ unabhängig voneinander für Sauerstoff, Schwefel oder die Gruppe $-N(CH_3)-$ stehen und m und n unabhängig voneinander eine ganze Zahl im Bereich von 1 bis 6 bedeuten und

$R^3$ und $R^4$ unabhängig voneinander eine $C_1$- bis $C_{10}$-Alkylgruppe, eine substituierte $C_1$- bis $C_{10}$-Alkylgruppe, eine Cycloalkylgruppe, eine gegebenenfalls substituierte Phenylgruppe, eine Alkoxy- und/oder Phenoxygruppe, eine Nitrogruppe und/oder Chlor,

wobei solche Dicarbonsäuren ausgenommen sind, die innere Anhydride bilden können.

Bevorzugte mehrbasische Carbonsäuren für das erfindungsgemäße Verfahren sind aromatische und heteroaromatische Ringe enthaltende Di- und Tricarbonsäuren, insbesondere Dicarbonsäuren, die bezüglich der Carboxylgruppen eine spiegelbildliche Molekülstruktur aufweisen und bei denen die Carboxylgruppen so angeordnet sind, daß die Bildung innerer Anhydride nicht möglich ist. Besonders bevorzugt sind Isophthalsäure und Terephthalsäure.

Die mehrbasischen Carbonsäuren können einzeln oder in beliebigen Gemischen untereinander eingesetzt werden. Von diesen Gemischen sind solche besonders bevorzugt, die aus Iso- und Terephthalsäure in beliebigen Verhältnissen bestehen.

Für das erfindungsgemäße Verfahren geeignete Anhydride einbasischer $C_2$- bis $C_4$-Carbonsäuren sind beispielsweise solche, die ein verzweigtes oder unverzweigtes Kohlenstoffgerüst enthalten und der Formel (VII) entsprechen

$$R-\overset{O}{\overset{\|}{C}}-O-\overset{O}{\overset{\|}{C}}-R' \qquad (VII)$$

in der R und R' unabhängig voneinander für einen geradkettigen oder verzweigten $C_1$- bis $C_3$-Alkylrest stehen.

Bevorzugt sind hier Propionsäureanhydrid und Essigsäureanhydrid, besonders bevorzugt das Essigsäureanhydrid.

Die Menge des Säureanhydrids einer einbasischen Carbonsäure wird im allgemeinen so bemessen, daß pro Mol umzusetzender Carboxylgruppen der mehrbasischen Carbonsäure(n) mindestens ein halbes Mol Säureanhydrid eingesetzt wird. Vorzugsweise wird das Säureanhydrid in einer Menge eingesetzt, die dem 1,05- bis 10-fachen, besonders bevorzugt dem 1,05- bis 4-fachen dieser Mindestmenge entspricht.

Die erste Stufe des erfindungsgemäßen Verfahrens zur Herstellung von Säurechloriden mehrbasischer Carbonsäuren, welche die Herstellung einer Suspension polymerer Anhydride von mehrbasischen Carbonsäuren umfaßt, wird im allgemeinen bei Normaldruck durchgeführt. Sie kann jedoch auch bei erniedrigtem oder erhöhtem Druck durchgeführt werden, beispielsweise bei 0,5 bis 10 bar. Die Temperatur für die erste Stufe des Verfahrens wird zumindest gegen Ende mindestens so hoch gewählt, daß die entstehende Monocarbonsäure und das gegebenenfalls vorhandene überschüssige Anhydrid der einbasischen $C_2$- bis $C_4$-Carbonsäure abdestilliert werden können. Beim Arbeiten bei Normaldruck kann die Temperatur beispielsweise im Bereich von 140 bis 220 °C liegen, vorzugsweise bei 150 bis 200 °C. Die entstehende Monocarbonsäure kann in dem Maße abdestilliert werden wie sie entsteht. Man kann auch zunächst das Reaktionsgemisch am Rückfluß kochen und erst nach einiger Zeit, z. B. nach 30 Minuten bis 4 Stunden, mit dem Abdestillieren der Monocarbonsäure beginnen. Gegen Ende der Umsetzung, je nach den Reaktionspartnern und den Reaktionsbedingungen ist das nach etwa 1 Stunde bis einem Tag, fällt nur noch wenig Monocarbonsäure an. Zusammen mit der Monocarbonsäure oder anschließend daran wird der gegebenenfalls vorhandene Überschuß des Anhydrids einer einbasischen $C_2$- bis $C_4$-Carbonsäure abdestilliert.

Für das Abdestillieren der Monocarbonsäure und des gegebenenfalls vorhandenen Überschusses am Anhydrid einer einbasischen $C_2$- bis $C_4$-Carbonsäure verwendet man vorzugsweise eine Destillationskolonne oder einen Dephlegmator, die man so betreibt, daß das Destillat möglichst hohe Anteile an Monocarbonsäure enthält.

Das einzusetzende Lösungsmittel muß folgende Kriterien erfüllen :

— Es muß unter den Verfahrensbedingungen gegenüber den Reaktionsteilnehmern weitestgehend inert sein.

— Es muß das eingesetzte Anhydrid einer einbasischen $C_2$- bis $C_4$-Carbonsäure zu lösen vermögen.

— Es muß höher sieden als das eingesetzte Anhydrid einer einbasischen $C_2$- bis $C_4$-Carbonsäure.

— Es darf die gebildeten polymeren Anhydride mehrbasischer Carbonsäuren allenfalls in geringen Mengen lösen.

Bevorzugt sind solche Lösungsmittel, die zusätzlich das gebildete Säurechlorid einer mehrbasischen Carbonsäure zu lösen vermögen und davon leicht abzutrennen sind.

Das zweite Ausgangsprodukt, die mehrbasischen Carbonsäuren, brauchen in dem verwendeten Lösungsmittel nicht löslich zu sein.

Geeignete Lösungsmittel, die den vorstehenden Kriterien genügen, sind beispielsweise geradkettige

und verzweigte Kohlenwasserstoffe mit 9 bis 20 C-Atomen wie Nonane, Decane, Undecane, Dodecane und Tetradecane, cyclische Kohlenwasserstoffe mit Siedepunkten über 140 °C wie Decalin, Tetralin, Trimethylbenzole, entsprechende Chlorkohlenwasserstoffe mit bis zu 4 Chloratomen pro Molekül wie Dichlorbenzole und Trichlorbenzole und Ether mit Siedepunkten über 140 °C wie Diethylenglykoldimethylether, Diethylenglykoldiethylether, Diphenylether, Anisol und Ethoxybenzol, die jeweils einzeln oder in beliebigen Mischungen untereinander eingesetzt werden können. Ebenso sind Kohlenwasserstofffraktionen mit genügend hohem Siedepunkt geeignet. Vorzugsweise wird als Lösungsmittel n-Decan, Decalin, Tetralin, m-Dichlorbenzol, p-Dichlorbenzol und/oder o-Dichlorbenzol eingesetzt.

Im allgemeinen ist es günstig, möglichst konzentriert zu arbeiten, d. h. möglichst viel mehrbasissche Carbonsäure pro Volumeneinheit umzusetzen. Ausführungsformen des erfindungsgemäßen Verfahrens, bei welchem die mehrbasischen Carbonsäuren ganz oder teilweise suspendiert vorliegen, sind deshalb bevorzugt.

Das Lösungsmittel wird vorzugsweise in einer solchen Menge verwendet, daß das Reaktionsgemisch gut rührbar ist. Die Lösungsmittelmenge kann in den einzelnen Reaktionsstufen variieren, z. B. indem Lösungsmittel hinzugefügt oder abdestilliert wird. Im allgemeinen beträgt der Anteil des Lösungsmittels mehr als 20 Gew.-%, bezogen auf das gesamte Reaktionsgemisch. Vorzugsweise liegt die Lösungsmittelmenge im Bereich von 20 bis 70 Gew.-%, bezogen auf das gesamte Reaktionsgemisch. Im ersten Reaktionsschritt sind jedoch auch Ausführungsformen des erfindungsgemäßen Verfahrens günstig, bei welchen zu Beginn der Umsetzung noch kein Lösungsmittel vorliegt, sondern erst im Laufe der Umsetzung, die unter Abdestillieren von Monocarbonsäure erfolgt, Lösungsmittel hinzugefügt wird, vorzugsweise in einer solchen Menge, daß das Volumen des Reaktionsgemisches sich nicht wesentlich ändert.

Die zweite Stufe des erfindungsgemäßen Verfahrens zur Herstellung von Säurechloriden, die Umsetzung mit Phosgen, erfolgt im allgemeinen direkt in der Suspension, die nach der ersten Reaktionsstufe vorliegt.

Das in der zweiten Stufe einzusetzende Phosgen kann in technisch üblicher Spezifikation verwendet werden, wie sie beispielsweise zur Herstellung von Isocyanaten und Polycarbonaten einzuhalten ist. Das Phosgen kann auch im Gemisch mit Inertgasen, wie Kohlendioxid oder Stickstoff, verwendet werden. Es kann auch geringe Mengen Chlorwasserstoff enthalten. Ebenso ist es möglich, das Phosgen in gelöster Form, beispielsweise gelöst in Methylenchlorid, Chlorbenzol oder Dichlorbenzol zu verwenden.

Die Menge des Phosgens wird im allgemeinen so bemessen, daß pro Mol der in den eingesetzten mehrbasischen Carbonsäuren vorhandenen Carboxylgruppen mindestens ein halbes Mol Phosgen zur Verfügung steht, beispielsweise also im Falle von Terephthalsäure im allgemeinen mindestens 1 Mol Phosgen pro Mol Terephthalsäure verwendet wird. Das Phosgen kann in beliebig hohem Überschuß verwendet werden, jedoch ist es aus Gründen der Wirtschaftlichkeit und der Entsorgung nicht zweckmäßig mehr als das Doppelte dieser Mindestmenge an Phosgen einzusetzen.

Besonders vorteilhaft ist es, das den Reaktor verlassende Gas zurückzuführen, um so die Phosgenausnutzung zu erhöhen. Hierdurch ist es im allgemeinen möglich, mit der 1,05- bis 1,3-fachen der Mindestmenge an Phosgen auszukommen.

Zur Aktivierung des Phosgens werden Katalysatoren verwendet, beispielsweise organische Phosphor- und/oder Stickstoffverbindungen. Solche Verbindungen werden eingesetzt, um die Reaktionszeiten zu verkürzen, d. h. um die Verfahrensprodukte in technisch günstigen Raum-Zeit-Ausbeuten zu gewinnen.

Beispiele für Katalysatoren sind Phosphine, Phosphinoxide, cyclische Azaverbindungen, Amine und Carboxamide. Solche katalytisch wirksame Verbindungen sind im Prinzip bekannt und beispielsweise beschrieben in : EP-OS 0 050 783, EP-OS 0 050 775, EP-OS 0 021 373, US-PS 3 318 950, DE-OS 2 400 007, EP-OS 0 027 941, DE-OS 2 933 148, DE-OS 2 926 789, DE-OS 2 926 779, DE-OS 2 321 122, US-PS 3 547 960, US-PS 3 544 627, US-PS 3 544 626 und US-PS 3 184 506.

Bevorzugte Katalysatoren sind Trialkylphosphine, Triarylphosphine, Carboxamide, cyclische Azaverbindungen und tertiäre Amine, jeweils im Molekulargewichtsbereich bis etwa 500. Besonders bevorzugt sind Triarylphosphine, N,N-Dialkylcarboxamide, tertiäre Amine, insbesondere cyclische Azaverbindungen mit trisubstituiertem Stickstoff. Ganz besonders bevorzugt sind Triphenylphosphin, Pyridin, Chinolin, Isochinolin und N,N-Dimethylformamid.

Auch Gemische der obengenannten Verbindungen sind als Katalysatoren geeignet, ebenso Verbindungen, die zwei oder mehrere dreifach substituierte Phosphor- und/oder Stickstoff-Atome pro Molekül aufweisen, wie Bis-(1,2-diphenyl-phosphino)-benzol und Tris(4-dimethylaminophenyl)-phosphin. Polymere, die tertiäres Amin und/oder Phosphin als Strukturelement enthalten, beispielsweise Polyvinylpyridin oder polymer gebundenes Triphenylphosphin (siehe US-PS 3 708 462) können, abweichend von der oben erwähnten Molekulargewichtsbeschränkung, ebenfalls als Katalysatoren verwendet werden. Solche polymeren Katalysatoren sind für das erfindungsgemäße Verfahren ebenfalls bevorzugt.

Die Katalysatoren werden im allgemeinen in einer Menge von 0,01 bis 10 Gew.-% verwendet, vorzugsweise in einer Menge von 0,2 bis 5 Gew.-%, jeweils bezogen auf das gesamte Reaktionsgemisch zu Beginn des Phosgenierschrittes. Werden Katalysatoren verwendet, die ausschließlich dreifach substituierte Stickstoff- und/oder Phosphor-Atome als katalytisch wirksames Strukturelement aufweisen, so können diese bereits in der ersten Reaktionsstufe zugegen sein, ohne daß diese beeinträchtigt wird.

Als Lösungsmittel für die zweite Reaktionsstufe kommen diejenigen in Betracht, die oben für die erste Reaktionsstufe beschrieben wurden. Es ist vorteilhaft, beide Reaktionsstufen in Gegenwart des gleichen Lösungsmittels durchzuführen. Hierdurch ist es möglich, die Säurechloride nach einem Eintopfverfahren absatzweise herzustellen oder bei kontinuierlicher Arbeitsweise ohne zwischengeschaltete Trennschritte beide Reaktionsstufen unmittelbar hintereinander durchzuführen.

Die zweite Stufe des erfindungsgemäßen Verfahrens zur Herstellung von Säurechloriden kann beispielsweise bei Temperaturen im Bereich von 80 bis 200 °C durchgeführt werden. Bevorzugt sind hier Temperaturen im Bereich von 120 bis 170 °C.

Zur besseren Nutzung des Phosgens kann man den entweichenden Gasstrom ganz oder teilweise zurückführen. Man kann aus dem entweichenden Gasstrom auch das dort noch vorhandene Phosgen rückgewinnen, beispielsweise durch Auswaschen oder ein Absorptionsverfahren und das rückgewonnene Phosgen erneut einsetzen. Besonders vorteilhaft ist es, das Phosgen mit dem bei der Reaktion verwendeten Lösungsmittel aus dem Abgasstrom auszuwaschen und das phosgenbeladene Lösungsmittel zurückzuführen.

Die zu Beginn der zweiten Reaktionsstufe vorliegende Suspension geht im allgemeinen im Laufe der Phosgenbehandlung in eine Lösung über. Das Einleiten von Phosgen kann im allgemeinen dann beendet werden, wenn die Suspension klar wird oder kurz danach, da dann im allgemeinen die Umsetzung zum gewünschten Säurechlorid beendet ist. Vor der weiteren Aufarbeitung ist es zweckmäßig, das Rohprodukt durch Durchleiten eines Inertgases, z. B. Stickstoff, und/oder durch Anlegen eines leichten Vakuums von restlichem Phosgen zu befreien.

Die Aufarbeitung des Rohproduktes kann nach üblichen Methoden erfolgen, z. B. durch Destillation, Rektifikation, Extraktion und/oder Kristallisation in kontinuierlichen und/oder diskontinuierlichen Ausführungsformen.

Häufig, insbesondere im Falle der besonders bevorzugt einzusetzenden mehrbasischen Carbonsäuren, ist es möglich das Reaktionsgemisch durch destillative Methoden aufzuarbeiten. Beispielsweise kann man in einer ersten Destillationsstufe das Lösungsmittel, evtl. zusammen mit dem Katalysator, bei Normaldruck oder mäßigem Vakuum, z. B. bei 1 020 bis 10 mbar abdestillieren.

Der Rückstand aus dieser Destillation kann dann einer Rektifikation im Druckbereich von beispielsweise 0,1 bis 50 mbar unter Verwendung einer Destillationskolonne, vorzugsweise einer Bodenkolonne, unterworfen werden. Der Sumpf der Rektifikation enthält dann gegebenenfalls den Katalysator oder ein ebenfalls aktives Folgeprodukt des Katalysators. Der Katalysator oder sein Folgeprodukt kann aus dem Sumpfprodukt abgetrennt und zurückgeführt werden. Man kann auch den größeren Teil des katalysatorhaltigen Sumpfproduktes direkt in die zweite Reaktionsstufe zurückführen.

Im allgemeinen ist das Kopfprodukt der Rektifikation nach Abnahme eines Vorlaufs, der noch Lösungsmittelreste enthält, ein hochreines polykondensationsfähiges Säurechlorid der eingesetzten mehrbasischen Carbonsäure. Es kann in dieser Qualität unter Ausnutzung rückgeführter Seitenströme in Gesamtausbeuten von beispielsweise 80 bis 96 Mol-%, bezogen auf eingesetzte mehrbasische Carbonsäure erhalten werden.

Bei der destillativen Aufarbeitung zurückgewonnenes Lösungsmittel kann in die erste Reaktionsstufe zurückgeführt werden. Sollte das durch Destillation zurückgewonnene Lösungsmittel außerdem noch Katalysator enthalten, so kann es in der Regel ohne weitere Auftrennung ebenfalls in die erste Reaktionsstufe zurückgeführt werden, da der Katalysator dort im allgemeinen nicht stört.

Die aus der ersten Reaktionsstufe abdestillierte Monocarbonsäure oder Gemische von Monocarbonsäuren mit Anhydriden einbasischer $C_2$- bis $C_4$-Carbonsäuren können wieder zur Herstellung dieser Anhydride, vorzugsweise nach einem Pyrolyseverfahren, verwendet werden. Es ist auch möglich, destillativ abgetrennte Monocarbonsäure anderweitig zu verwenden.

Die vorliegende Erfindung betrifft weiterhin ein Verfahren zur Herstellung von polymeren Anhydriden mehrbasischer Carbonsäuren durch Umsetzung von mehrbasischen Carbonsäuren, die mindestens zwei nicht zu einer inneren Anhydridbildung befähigte Carboxylgruppen enthalten, mit Anhydriden einbasischer $C_2$- bis $C_4$-Carbonsäuren, das dadurch gekennzeichnet ist, daß man die Umsetzung bei 140 bis 220 °C und 0,5 bis 10 bar durchführt, vor oder während der Umsetzung ein Lösungsmittel zusetzt, in dem die Anhydride einbasischer $C_2$- bis $C_4$-Carbonsäuren löslich sind, das höher siedet als diese und in dem die polymeren Anhydride mehrbasischer Carbonsäuren unlöslich sind, wobei man aus dem Reaktionsgemisch die niedriger als das Lösungsmittel siedenden Bestandteile abtrennt und abschließend das Lösungsmittel entfernt.

Einzelheiten zu diesem Verfahren zur Herstellung von polymeren Anhydriden mehrbasischer Carbonsäuren sind vorstehend als erste Stufe des erfindungsgemäßen Verfahrens zur Herstellung von Säurechloriden mehrbasischer Carbonsäuren beschrieben. Um zu reinen polymeren Anhydriden mehrbasischer Carbonsäuren zu gelangen sind diese lediglich aus der nach der Abtrennung der niedriger als das Lösungsmittel siedenden Bestandteilen vorliegenden Suspension zu isolieren, indem man das Lösungsmittel entfernt. Dies kann beispielsweise durch mechanische Abtrennung, Nachwaschen mit einem niedriger siedenden Lösungsmittel und Trocknung bei erhöhter Temperatur geschehen.

Schließlich betrifft die vorliegende Erfindung Suspensionen von polymeren Anhydriden mehrbasischer Carbonsäuren, die 30 bis 80 Gew.-% polymere Anhydride mehrbasischer Carbonsäuren und ergänzend zu 100 Gew.-% eines oder mehrere Lösungsmittel aus der Gruppe geradkettiger und

verzweigter Kohlenwasserstoffe mit 9 bis 20 C-Atomen, cyclischer Kohlenwasserstoffe oder entsprechender Chlorkohlenwasserstoffe mit bis zu 4 Chloratomen pro Molekül und Ether, jeweils mit Siedepunkten über 140 °C, enthalten. Die Herstellung solcher Suspension ist vorstehend als erste Stufe des erfindungsgemäßen Verfahrens zur Herstellung von Säurechloriden mehrbasischer Carbonsäuren beschrieben worden.

Solche Suspensionen können beispielsweise zur Herstellung von Säurechloriden mehrbasischer Carbonsäuren verwendet werden, indem man sie, wie oben beschrieben, mit Phosgen in Gegenwart von Katalysatoren umsetzt.

Die auf erfindungsgemäße Weise hergestellten Säurechloride mehrbasischer Carbonsäuren sind von sehr hoher Reinheit und hervorragend geeignet, um daraus Polykondensate herzustellen, beispielsweise durch Umsetzung mit Bisphenol A. Die erfindungsgemäß hergestellten Säurechloride sind beispielsweise so rein, daß sie zur Herstellung von farblosen Polyestern nach dem in der DE-OS 3 346 946 beschriebenen Verfahren verwendet werden können. Als Nebenprodukte fallen im erfindungsgemäßen Verfahren praktisch nur $C_2$- bis $C_4$-Monocarbonsäuren und Kohlendioxid an. Die Monocarbonsäuren können, wie beschrieben, weiterverwendet werden. Die Entsorgung von Kohlendioxid bereitet keine Probleme. Das Verfahren ist auf einfache Weise durchführbar und für die Herstellung technischer Mengen an Säurechloriden mehrbasischer Carbonsäuren gut geeignet. Hohe Temperaturen und niedrige Drucke können vermieden werden. Es ist von besonderem Vorteil, daß man die eingesetzten mehrbasischen Carbonsäuren und die daraus in der ersten Stufe entstehenden polymeren Anhydride nicht in gelöster Form umsetzen muß, denn gerade so wichtige Säurechloride, wie Terephthalsäuredichlorid, leiten sich von Säuren ab, die in üblichen Lösungsmitteln nur schlecht oder garnicht löslich sind. Die als Zwischenprodukte auftretenden polymeren Anhydride sind ebenfalls in üblichen Lösungsmitteln unlöslich. Gemäß dem Stand der Technik wurde es aber bisher als erforderlich gehalten, die polymeren Anhydride bzw. die Anhydride enthaltenden Verbindungen in Lösung, z. B. in rückgeführtem Carbonsäurechlorid, mit Phosgen umzusetzen.

Das erfindungsgemäße Verfahren zur Herstellung von polymeren Anhydriden mehrbasischer Carbonsäuren ist den Verfahren des Standes der Technik überlegen, weil auf einfachere Weise und aus gut zugänglichen Ausgangsprodukten Produkte mit hohen Molekulargewichten erhalten werden, wobei keine störenden Nebenprodukte entstehen.

Die erfindungsgemäßen Suspensionen, die polymere Anhydride mehrbasischer Carbonsäuren enthalten sind neu und als solche Zwischenprodukte zur Herstellung von Säurechloriden mehrbasischer Carbonsäuren.

Die mit der vorliegenden Erfindung erzielbaren Fortschritte sind ausgesprochen überraschend, denn gemäß der US-PS 3 318 950 wird für den Phosgenierschritt ausdrücklich von fest/flüssig/gasförmigen Reaktionssystemen abgeraten.

Die nachstehenden Beispiele illustrieren die erfindungsgemäßen Verfahren und die erfindungsgemäße Suspension, ohne die Erfindung auf diese Ausführungsformen zu beschränken.

## Beispiele

### Allgemeines

Die verwendete Iso- und Terephthalsäure war handelsübliche Ware von etwa 98,5 %iger Reinheit. Das Acetanhydrid wurde in technischer Qualität undestilliert eingesetzt, das ortho-Dichlorbenzol wurde ebenfalls in technischer Qualität verwendet, jedoch vor dem Gebrauch destilliert.

Die Reinheitsbestimmungen der Säuredichloride erfolgte gaschromatographisch, im Spurenbereich unter Verwendung einer Glaskappilare nach der on-column-Technik.

### Beispiel 1

In einer 5 l-Rührapparatur aus Glas mit Doppelmantel zur Beheizung mit einem Ölumlaufthermostaten und mit Destillationsaufsatz (150 cm Füllkörperkolonne) wurden 1 079 g (6,5 Mol) Isophthalsäure und 1 079 g (6,5 Mol) Terephthalsäure in feinpulveriserter Form in 2 918 g (28,6 Mol) Acetanhydrid eingerührt und 3 Stunden auf Rückfluß erhitzt. Das Rühren wurde während der gesamten Reaktion in der ersten und zweiten Reaktionsstufe fortgesetzt. Danach wurde ein Gemisch von Essigsäure und Acetanhydrid über Kopf abgenommen und, etwa volumengleich zum anfallenden Destillat, ortho-Dichlorbenzol zugetropft (insgesamt 2 050 g). Nach zwei weiteren Stunden war die erste Reaktionsstufe beendet, erkenntlich daran, daß die Siedetemperatur auf den Siedepunkt des ortho-Dichlorbenzols anstieg. Während der gesamten ersten Reaktionsstufe lag ein weißer suspendierter Feststoff vor.

Zu Beginn der zweiten Reaktionsstufe wurde auf 150 °C abgekühlt, 40 g Pyridin hinzugefügt und die Zu- und Ableitung für Phosgen angeschlossen. Während des Einleitens von Phosgen lösten sich die Feststoffpartikel im Laufe von 5 Stunden auf. Es entstand eine braune Lösung und das Phosgeneinleiten wurde beendet. Der Gesamtverbrauch an Phosgen im geraden Durchgang, d. h. ohne Rückführung betrug 2 380 g = 185 % der Theorie.

Danach wurde bei 20 mbar ortho-Dichlorbenzol und Pyridin aus der rohen Reaktionslösung am

Rotationsverdampfer (Badtemperatur bis 80 °C) weitgehend abgezogen. Der Rückstand (2 655 g) wurde bei 0,5 mbar über eine 70 cm Siebbodenkolonne mit 20 praktischen Böden destilliert. Bei 95-98 °C Kopftemperatur wurde nach einem Vorlauf die Hauptfraktion abgenommen. Es wurden so 2 243,5 g (= 85 % der Theorie) eines Isomerengemisches aus Iso- und Terephthalsäuredichlorid im Verhältnis 48,5/51,6 Gew.-Teile von 99,992 %iger Reinheit (bezüglich der Säurechloride) erhalten. Der Vorlauf (141 g) und der Sumpf (183,5 g) enthielten jeweils noch über 90 Gew.-% an Säuredichloriden.

Beispiel 2

Es wurden 1 079 g (6,5 Mol) Isophthalsäure und 1 079 g (6,5 Mol) Terephthalsäure feinpulverisiert in 2 300 g o-Dichlorbenzol in der Apparatur von Beispiel 1 vorgelegt. Bei 160 bis 170 °C (gemessen in der Suspension) wurden unter Rühren innerhalb von 6 Stunden 2 570 g (25,2 Mol) Acetanhydrid zugetropft und gleichzeitig bei 119 bis 130 °C Kopftemperatur ein Gemisch von Essigsäure und Acetanhydrid abdestilliert. Danach wurde die Temperatur gesteigert, bis sich am Kolonnenkopf die Siedetemperatur von ortho-Dichlorbenzol (179 °C) einstellte.

Die zweite Reaktionsstufe wurde wie in Beispiel 1 angegeben durchgeführt. Man erhielt im Hauptlauf der Destillation Iso/Terephthalsäurechlorid in 82 % Ausbeute und 99,99 %iger Reinheit.

Beispiele 3 bis 9

Es wurde wie in Beispiel 1 gearbeitet, jedoch wurden anstelle von Pyridin andere Katalysatoren verwendet. Die Ergebnisse sind aus Tabelle 1 ersichtlich.

(Siehe Tabelle 1 Seite 9 f.)

Tabelle 1

| Beispiel Nr. | Katalysator Bezeichnung | Katalysator Menge (g) | Hauptfraktion Ausbeute (%) | Reinheit (%) Iso/Terephthal-säuredichlorid |
|---|---|---|---|---|
| 3 | Chinolin | 40 | 83 | 99,98 |
| 4 | N,N-Diethyl-anilin | 40 | 85 | 99,95 |
| 5 | N,N-Dimethyl-formamid | 40 | 80 | 99,96 |
| 6 | 1,4-Diaza-(2,2,2)-bicyclooctan | 20 | 78 | 99,98 |
| 7 d) | a) | 20 | 83 b) | 99,98 |
| 8 | Triphenyl-phosphin | 20 | 86 | 99,98 |
| 9 d) | c) | 20 | 82 b) | 99,98 |

a) Copolymer aus Vinylpyridin und 10 % Styrol ; Jansen, Bestell-Nr. : 19 204-4
b) Nach der Phosgenbehandlung wurde der polymere Katalysator abfiltriert
c) Triphenylphosphin auf vernetztem Polystyrol (2 % Divinylbenzol) ; Alfa, Bestell-Nr. : 18579
d) Die Behandlung mit Phosgen wurde bei 120 °C durchgeführt.

Beispiel 10

Es wurde wie in Beispiel 1 gearbeitet, jedoch wurden statt des Säuregemisches 13 Mol Terephthalsäure eingesetzt. Terephthalsäuredichlorid wurde in der Hauptfraktion mit 81 % Ausbeute (Kp. 93-96 °C bei 0,5 mbar) in 99,985 %iger Reinheit erhalten.

Beispiel 11

Es wurde wie in Beispiel 1 gearbeitet, jedoch wurden statt des Säuregemisches 13 Mol Isophthalsäure eingesetzt. Isophthalsäuredichlorid wurde in der Hauptfraktion mit 84 % Ausbeute in 99,988 %iger Reinheit erhalten.

Beispiele 12-14

Zur Isolierung der polymeren Anhydride, die Zwischenprodukte des erfindungsgemäßen Verfahrens darstellen, wurde die erste Reaktionsstufe wie in den Beispielen 1, 10 und 11 angegeben durchgeführt. Die so erhaltene Suspension der polymeren Anhydride wurde auf 100 °C abgekühlt und bei dieser Temperatur abfiltriert. Der Filterkuchen wurde mit n-Hexan gewaschen, dann in siedendem n-Hexan aufgerührt. Nach dem Abfiltrieren wurde der Filterrückstand im Umluftschrank bei 100 °C getrocknet und nach der DSC-Methode untersucht. Die Ergebnisse sind aus Tabelle 2 ersichtlich.

(Siehe Tabelle 2 Seite 11 f.)

Tabelle 2

| Beispiel | Polym. Anhydrid isoliert nach der 1. Stufe von Beispiel | Tg [1] °C | Schmelzvorgang °C [2] $T_1$ | $T_{max}$ | $T_2$ | Röntgenographischer Befund |
|---|---|---|---|---|---|---|
| 12 | 1 | 113 | 220 | 280 | 325 | teilkristallin |
| 13 | 10 | - | 425 | 455[3] | - | praktisch vollständig kristallin |
| 14 | 11 | 108 | 250 | 280 | 285 | weitgehend kristallin |

1) Glasübergangstemperatur

2) $T_1$ : Beginn, $T_{max}$ : Temperatur der maximalen Enthalpieänderung,

$T_2$ : Endtemperatur des Schmelzvorganges

3) Zersetzung

Henglein und Tarrach (a.a.O.) geben für Polyterephthalsäureanhydride mit den Polymerisationsgraden 25 und 33 Zersetzungstemperaturen von 438 und 445 °C an. Durch Extrapolation von Schmelzpunkten gemischter Polyanhydride auf 100 %iges Polyterephthalsäureanhydrid ist nach N. Yoda und A. Miyake, Bull. Soc. Chim. Jap. 32 (1959), 1120-1126 ein Schmelzpunkt von 410 °C zu erwarten.

**Patentansprüche**

1. Verfahren zur Herstellung von Säurechloriden mehrbasischer Carbonsäuren, dadurch gekennzeichnet, daß man zunächst aus mehrbasischen Carbonsäuren, die mindestens zwei nicht zu einer inneren Anhydridbildung befähigte Carboxylgruppen enthalten, durch Umsetzung mit Anhydriden einbasischer $C_2$- bis $C_4$-Carbonsäuren polymere Anhydride mehrbasischer Carbonsäuren herstellt, wobei man vor oder während dieser Reaktion ein Lösungsmittel zusetzt, in dem die Anhydride einbasischer $C_2$- bis $C_4$-Carbonsäuren löslich sind, das höher siedet als diese und in dem die polymeren Anhydride mehrbasischer Carbonsäuren unlöslich sind, und anschließend in die nach Abtrennung niedriger als das Lösungsmittel siedender Bestandteile erhaltene Suspension Phosgen in Gegenwart von Katalysatoren einleitet.

2. Verfahren nach Anspruch 1, dadurch gekennzeichnet, daß man als mehrbasische Carbonsäuren solche einsetzt, die ein aliphatisches, cycloaliphatisches, heterocyclisches, aromatisches oder araliphatisches Gerüst mit 4 bis 50 C-Atomen enthalten.

3. Verfahren nach Ansprüchen 1 und 2, dadurch gekennzeichnet, daß man als mehrbasische Carbonsäuren Adipinsäure, Sebacinsäure, cis-1,4-Cyclohexandicarbonsäure, trans-1,4-Cyclohexandicarbonsäure, Trimesinsäure, Isophthalsäure, Terephthalsäure, 2,5-Furandicarbonsäure und/oder Dicarbonsäuren, die den Formelm (I) bis (VI) entsprechen

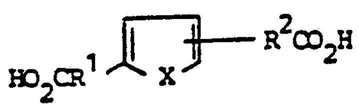

(I)

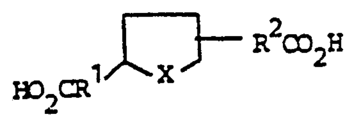

(II)

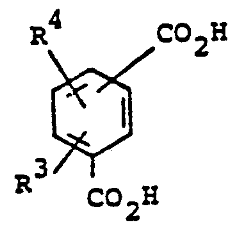

(III)

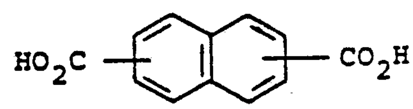

(IV)

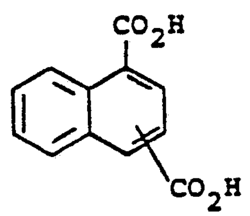

(V)

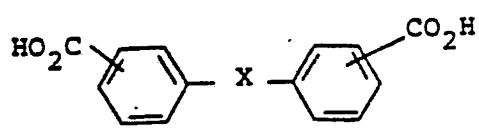

(VI)

in denen

X Sauerstoff, Schwefel oder die Gruppe $-N(CH_3)-$,

$R^1$ und $R^2$ unabhängig voneinander eine Einfachbindung, eine $C_1$- bis $C_6$-Alkylengruppe, ein $-CO-$ oder $-SO_2-$Gruppe oder die Gruppe $-X_1-(CH_2)_m-X_2-(CH_2)_n-X_3-$, in der

$X_1$, $X_2$ und $X_3$ unabhängig voneinander für Sauerstoff, Schwefel oder die Gruppe $-N(CH_3)-$ stehen und m und n unabhängig voneinander eine ganze Zahl im Bereich von 1 bis 6 bedeuten und

$R^3$ und $R^4$ unabhängig voneinander eine $C_1$ bis $C_{10}$-Alkylgruppe, eine substituierte $C_1$- bis $C_{10}$-Alkylgruppe, eine Cycloalkylgruppe, eine gegebenenfalls substituierte Phenylgruppe, eine Alkoxy- und/oder Phenoxygruppe, eine Nitrogruppe und/oder Chlor,

bedeuten, einsetzt,

wobei solche Dicarbonsäuren ausgenommen sind, die innere Anhydride bilden können.

4. Verfahren nach Ansprüchen 1 bis 3, dadurch gekennzeichnet, daß man pro Mol umzusetzender Carboxylgruppen der mehrbasischen Carbonsäure(n) 0,5 bis 10 Mol eines Anhydrids einer einbasischen $C_2$- bis $C_4$-Carbonsäure einsetzt.

5. Verfahren nach Ansprüchen 1 bis 4, dadurch gekennzeichnet, daß man als Lösungsmittel geradkettige oder verzweigte Kohlenwasserstoffe mit 9 bis 20 C-Atomen, cyclische Kohlenwasserstoffe oder entsprechende Chlorkohlenwasserstoffe mit bis zu 4 Chloratomen pro Molekül oder Ether, jeweils mit Siedepunkten über 140 °C, alleine oder in Mischungen untereinander einsetzt.

6. Verfahren nach Ansprüchen 1 bis 5, dadurch gekennzeichnet, daß man pro Mol der in der

eingesetzten mehrbasischen Carbonsäuren vorhandenen Carboxylgruppen 0,5 bis 1 Mol Phosgen einsetzt.

7. Verfahren nach Ansprüchen 1 bis 6, dadurch gekennzeichnet, daß man als Katalysatoren Phosphine, Phosphinoxide, cyclische Azaverbindungen, Amine oder Carboxamide, jeweils alleine oder in Mischungen untereinander, in einer Menge von 0,01 bis 10 Gew.-%, bezogen auf das gesamte Reaktionsgemisch zu Beginn des Phosgenierschrittes, einsetzt.

8. Verfahren nach Ansprüchen 1 bis 7, dadurch gekennzeichnet, daß man die Umsetzung mit dem Anhydrid einer einbasischen $C_2$- bis $C_4$-Carbonsäure bei 140 bis 220 °C und die Umsetzung mit Phosgen bei 80 bis 200 °C durchführt.

9. Verfahren zur Herstellung von Polymeren Anhydriden mehrbasischer Carbonsäuren durch Umsetzung von mehrbasischen Carbonsäuren, die mindestens zwei nicht zu einer inneren Anhydridbildung befähigte Carboxylgruppen enthalten, mit Anhydriden einbasischer $C_2$- bis $C_4$-Carbonsäuren, dadurch gekennzeichnet, daß man die Umsetzung bei 140 bis 220 °C und 0,5 bis 10 bar durchführt, vor oder während der Umsetzung ein Lösungsmittel zusetzt, wobei man aus dem Reaktionsgemisch die niedriger als das Lösungsmittel siedenden Bestandteile abtrennt und abschließend das Lösungsmittel entfernt.

10. Suspensionen von polymeren Anhydriden mehrbasischer Carbonsäuren, die 30 bis 80 Gew.-% polymere Anhydride mehrbasischer Carbonsäuren und ergänzend zu 100 Gew.-% eines oder mehrere Lösungsmittel aus der Gruppe geradkettiger und verzweigter Kohlenwasserstoffe mit 9 bis 20 C-Atomen, cyclischer Kohlenwasserstoffe oder entsprechender Chlorkohlenwasserstoffe mit bis zu 4 Chloratomen pro Molekül und Ether, jeweils mit Siedepunkten über 140 °C, enthalten.

**Claims**

1. Process for the preparation of acid chlorides of polybasic carboxylic acids, characterized in that polymeric anhydrides of polybasic carboxylic acids are first prepared from polybasic carboxylic acids, which contain at least two carboxyl groups which are not capable of forming an inner anhydride, by reaction with anhydrides of monobasic $C_2$- to $C_4$-carboxylic acids, a solvent in which the anhydrides of monobasic $C_2$- to $C_4$-carboxylic acids are soluble, which has a higher boiling point than these anhydrides and in which the polymeric anhydrides of polybasic carboxylic acids are insoluble being added before or during this reaction, and phosgene is then passed, in the presence of catalysts, into the suspension obtained after the constituents which have a lower boiling point than the solvent have been removed.

2. Process according to Claim 1, characterized in that the polybasic carboxylic acids employed are those which contain an aliphatic, cycloaliphatic, heterocyclic, aromatic or araliphatic skeleton with 4 to 50 C atoms.

3. Process according to Claims 1 and 2, characterized in that the polybasic carboxylic acids are adipic acid, sebacic acid, cis-1,4-cyclohexanedicarboxylic acid, trans-1,4-cyclohexanedicarboxylic acid, trimellitic acid, isophthalic acid, terephthalic acid, 2,5-furanedicarboxylic acid and/or dicarboxylic acids which correspond to the formulae (I) to (VI)

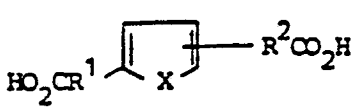

(I)

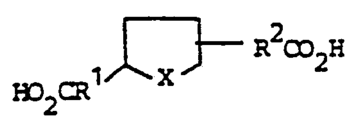

(II)

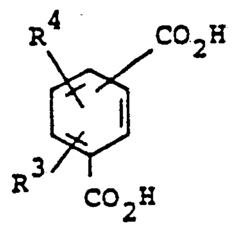

(III)

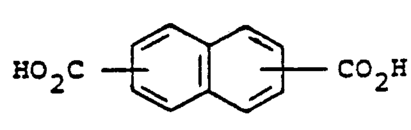

(IV)

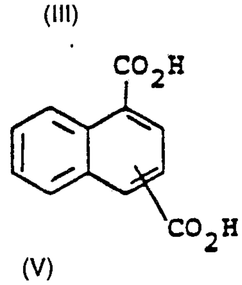

(V)

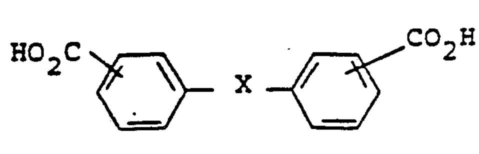

(VI)

in which

X denotes oxygen, sulphur or the group —$N(CH_3)$—, $R^1$ and $R^2$ independently of one another denote a single bond, a $C_1$- to $C_6$-alkylene group, a —CO— or —$SO_2$— group or the group —$X_1$—$(CH_2)_m$—$X_2$—$(CH_2)_n$—$X_3$—,

in which

$X_1$, $X_2$ and $X_3$ independently of one another represent oxygen, sulphur or the group —$N(CH_3)$— and m and n independently of one another denote an integer in the range from 1 to 6, and

$R^3$ and $R^4$ independently of one another denote a $C_1$- to $C_{10}$-alkyl group, a substituted $C_1$- to $C_{10}$-alkyl group, a cycloalkyl group, an optionally substituted phenyl group, an alkoxy and/or phenoxy group, a nitro group and/or chlorine,

those dicarboxylic acids which can form inner anhydrides being excluded.

4. Process according to Claims 1 to 3, characterized in that 0.5 to 10 moles of an anhydride of a monobasic $C_2$- to $C_4$-carboxylic acid are employed per mole of carboxyl groups to be reacted in the polybasic carboxylic acid(s).

5. Process according to Claims 1 to 4, characterized in that the solvents employed are straight-chain or branched hydrocarbons with 9 to 20 C atoms, cyclic hydrocarbons or corresponding chlorohydrocarbons with up to 4 chlorine atoms per molecule or ethers, in each case with boiling points above 140 °C, by themselves or as mixtures with one another.

6. Process according to Claims 1 to 5, characterized in that 0.5 to 1 mole of phosgene is employed per mole of the carboxyl groups present in the polybasic carboxylic acids employed.

7. Process according to Claims 1 to 6, characterized in that the catalysts employed are phosphines, phosphine oxides, cyclic aza compounds, amines or carboxamides, in each case by themselves or as mixtures with one another, in an amount of 0.01 to 10 % by weight, based on the total reaction mixture at the start of the phosgenation step.

8. Process according to Claims 1 to 7, characterized in that the reaction with the anhydride of a monobasic $C_2$- to $C_4$-carboxylic acid is carried out at 140 to 220 °C and the reaction with phosgene is carried out at 80 to 200 °C.

9. Process for the preparation of polymer anhydrides of polybasic carboxylic acids by reaction of polybasic carboxylic acids, which contain at least two carboxyl groups which are not capable of forming an inner anhydride, with anhydrides of monobasic $C_2$- to $C_4$-carboxylic acids, characterized in that the reaction is carried out at 140 to 220 °C under 0.5 to 10 bar, a solvent is added before or during the reaction, the constituents with lower boiling points than the solvent being removed from the reaction mixture, and, finally, the solvent is removed.

10. Suspensions of polymeric anhydrides of polybasic carboxylic acids containing 30 to 80 % by weight of polymeric anhydrides of polybasic carboxylic acids and, to make up to 100 % by weight, one or more solvents from the group comprising straight-chain and branched hydrocarbons with 9 to 20 C atoms, cyclic hydrocarbons and corresponding chlorohydrocarbons with up to 4 chlorine atoms per molecule, and ethers, in each case with boiling points above 140 °C.

**Revendications**

1. Procédé de fabrication de chlorures d'acides d'acides carboxyliques polybasiques, caractérisé en ce que tout d'abord à partir d'acides carboxyliques polybasiques qui contiennent au moins deux groupes carboxyle incapables de former un anhydride interne, on prépare par réaction avec des anhydrides d'acides carboxyliques monobasiques en $C_2$ à $C_4$ des anhydrides polymères d'acides carboxyliques polybasiques, on ajoute avant ou pendant cette réaction un solvant dans lequel les anhydrides d'acides carboxyliques monobasiques en $C_2$ à $C_4$ sont solubles, qui bout plus haut que ceux-ci et dans lequel les anhydrides polymères d'acides carboxyliques polybasiques sont insolubles et l'on fait passer ensuite, dans la suspension obtenue après séparation des constituants bouillant plus bas que le solvant, du phosgène en présence de catalyseurs.

2. Procédé selon la revendication 1, caractérisé en ce qu'on utilise comme acides carboxyliques polybasiques ceux qui contiennent un squelette aliphatique, cycloaliphatique, hétérocyclique, aromatique ou araliphatique ayant 4 à 50 atomes de carbone.

3. Procédé selon les revendications 1 et 2, caractérisé en ce qu'on utilise comme acides carboxyliques polybasiques l'acide adipique, l'acide sébacique, l'acide cis-1,4-cyclohexanedicarboxylique, l'acide trans-1,4-cyclohexanedicarboxylique, l'acide trimésique, l'acide isophtalique, l'acide téréphtalique, l'acide 2,5-furannedicarboxylique et/ou des acides dicarboxyliques qui correspondent aux formules (I) à (VI) :

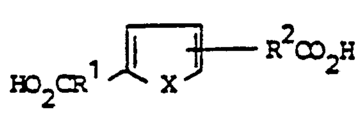

(I)

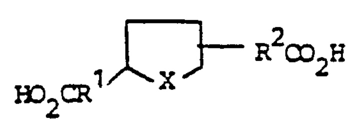

(II)

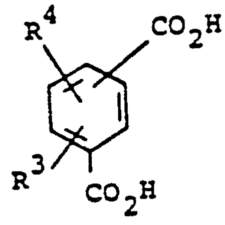

(III)

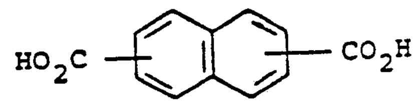

(IV)

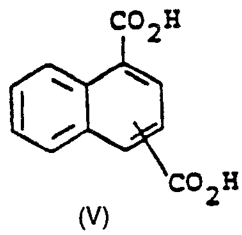

(V)

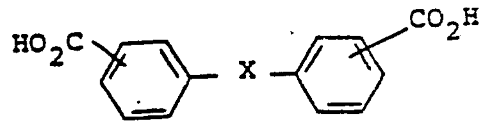

(VI)

dans lesquelles

X représente de l'oxygène, du soufre ou le groupe —N($CH_3$)—,

$R^1$ et $R^2$ indépendamment l'un de l'autre une simple liaison, un groupe alcoylène en $C_1$ à $C_6$, un groupe —CO— ou —$SO_2$— ou le groupe —$X_1$—$(CH_2)_m$—$X_2$—$(CH_2)_n$—$X_3$— où

$X_1$, $X_2$ et $X_3$ indépendamment les uns des autres représentent de l'oxygène, du soufre ou le groupe —N($CH_3$)— et m et n indépendamment l'un de l'autre un nombre entier dans l'intervalle de 1 à 6 et

$R^3$ et $R^4$ indépendamment l'un de l'autre signifient un groupe alcoyle en $C_1$ à $C_{10}$, un groupe alcoyle substitué en $C_1$ à $C_{10}$, un groupe cycloalcoyle, un groupe phényle éventuellement substitué, un groupe alcoxy et/ou phénoxy, un groupe nitro et/ou du chlore,

étant exceptés les acides dicarboxyliques qui peuvent former des anhydrides internes.

4. Procédé selon les revendications 1 à 3, caractérisé en ce que par mole de groupes carboxyle à faire réagir de(s) l'acide(s) carboxylique(s) polybasique(s), on utilise 0,5 à 10 moles d'un anhydride d'un acide carboxylique monobasique en $C_2$ à $C_4$.

5. Procédé selon les revendications 1 à 4, caractérisé en ce qu'on utilise comme solvant des hydrocarbures à chaîne droite ou ramifiée ayant 9 à 20 atomes de carbone, des hydrocarbures cycliques ou des hydrocarbures chlorés correspondants ayant jusqu'à 4 atomes de chlore par molécule, ou des éthers, tous ceux-ci ayant des points d'ébullition supérieurs à 140 °C, à eux seuls ou en des mélanges entre eux.

6. Procédé selon les revendications 1 à 5, caractérisé en ce que par mole des groupes carboxyle présents dans l'acide carboxylique polybasique mis en jeu on utilise 0,5 à 1 mole de phosgène.

7. Procédé selon les revendications 1 à 6, caractérisé en ce qu'on utilise comme catalyseurs des phosphines, des oxydes de phosphines, des azacomposés cycliques, des amines ou des carboxamides, chaque fois isolément ou en des mélanges entre eux, en une quantité de 0,01 à 10 % en poids par rapport au mélange de réaction tout entier au début du stade de phosgénation.

8. Procédé selon les revendications 1 à 7, caractérisé en ce qu'on exécute la réaction avec l'anhydride d'un acide carboxylique monobasique en $C_2$ à $C_4$ à 140 à 220 °C et la réaction avec le phosgène à 80 à 200 °C.

9. Procédé de fabrication d'anhydrides polymères d'acides carboxyliques polybasiques par réaction d'acides carboxyliques polybasiques qui contiennent au moins deux groupes carboxyle incapables de former un anhydride interne, avec des anhydrides d'acides carboxyliques monobasiques en $C_2$ à $C_4$, caractérisé en ce qu'on exécute la réaction à 140 à 220 °C sous 0,5 à 10 bars, on ajoute avant ou pendant la réaction un solvant, on sépare à partir du mélange de réaction les constituants bouillant plus bas que le solvant et on élimine pour finir le solvant.

10. Suspensions d'anhydrides polymères d'acides carboxyliques polybasiques qui contiennent 30 à 80 % en poids d'anhydrides polymères d'acides carboxyliques polybasiques et pour compléter à 100 % en poids un ou plusieurs solvants du groupe des hydrocarbures à chaîne droite ou ramifiée ayant 9 à 20 atomes de carbone, des hydrocarbures cycliques ou des hydrocarbures chlorés correspondants ayant jusqu'à 4 atomes de chlore par molécule, et des éthers, qui ont tous des points d'ébullition supérieurs à 140 °C.